# EUROPEAN PATENT SPECIFICATION

(11) **EP 0 963 555 B1**
(45) Date of publication and mention of the grant of the patent: **05.02.2003**
(21) Application number: 97947989.6
(22) Date of filing: 24.11.1997
(51) Int. Cl.: G01N 33/84, G01N 31/22, A01K 1/015

(54) **MEANS FOR DETERMINING THE DEGREE OF ACIDITY OF THE URINE OF A CAT**
MITTEL ZUR BESTIMMUNG DES SÄUREGRADES VON KATZENURIN
SYSTEME PERMETTANT DE MESURER LE TAUX D'ACIDITE DE L'URINE D'UN CHAT

(30) Priority: 29.11.1996 NL 1004646
(43) Date of publication of application: 15.12.1999
(73) Proprietor: Buchem Holding B.V., 7364AD Lieren (NL); Bumac B.V., 3891 KH Zeewolde (NL)
(72) Inventor: BUURMAN, Edward, NL-7364 AD Lieren (NL)
(74) Representative: Ferguson, Alexander
(86) International application number: NL9700642
(87) International publication number: WO98023966

(56) References cited:
- EP-A- 0 569 232
- WO-A-90/01695
- WO-A-93/15402
- GB-A- 922 606
- US-A- 3 791 794
- US-A- 3 978 818

## Description

The invention relates to means for determining the pH of a liquid, such as the urine of a cat, with the aid of colour change.

It is generally known that, by determining the pH or degree of acidity of the urine of a cat, it is possible to investigate whether the cat is suffering from the so-called Feline Urologic Syndrome (FUS). This is a disorder which occurs frequently in cats and it has been found that approximately 1 in 8 cats acquire this disorder in due course. This syndrome is characterized in that a large number of stones or crystals is formed in the urinary tract. As a result, the cat acquires problems with urinating and will attempt to refrain from urinating as a result of the partial blocking of the urethra and urinate only if the pressure becomes too great. Since the urination is accompanied with much pain, the cat will stop urinating as soon as the greatest pressure is released from the bladder. The cat will therefore regularly urinate small amounts, the urine often appearing bloody. In addition, because the cat puts off urinating, the risk of crystal formation occurring is only increased. The pH of the urine can be kept below 6.6 by dietary feeding, which level is decisive for whether crystal formation or stone formation occurs in the urinary tract or not. As soon as it is established that the cat has this disorder, the urine should be regularly examined since it is not always possible to investigate whether the cat is eating in addition to the diet, which can alter the pH.

The causes of the occurrence of the so-called FU syndrome are that the urine contains a high concentration of phosphate, magnesium and/or ammonium. With a high concentration of these substances, the formation of crystals may occur at a pH higher than 6.6 and the crystal formation will therefore be increased further if the cat urinates small amounts. The longer the urine is stored in the bladder, the greater the chance that crystal formation occurs and also the larger the crystals will become.

Attempts have already frequently been made to be able to determine the pH of the urine of a cat in a simple manner. One problem is generally that it is not possible to collect the urine of a cat since a cat does not in general permit this. A solution to this problem is to allow the cat to urinate regularly in the cat tray and to place indicators in the cat tray which give an indication of the degree of acidity of the urine of the cat.

Because a cat tray is usually filled at present with granules such as clay granules, it is possible to add to said clay granules granules to whose outside surface pH indicators have been applied, which indicators change colour at a certain value of the pH. From European Patent Application EP 0 569 232, it is known to provide certain granules with a layer into which one or more indicators have been introduced. It has been found, however, that as soon as these granules are moistened by the urine of the cat, elements which are contained in the interior of the granules dissolve in the urine, as well as elements from the granules which are in contact with the indicator granules. As a result, said dissolved elements will effect the determination of the degree of acidity of the urine, as a result of which the indicators do not give an exact indication of the pH or degree of acidity of the urine of the cat itself. Another drawback of this known procedure for determining the pH of the urine of a cat is that as soon as the surface of a granule provided with a layer containing one or more indicators is moistened, said layer will dissolve partially or completely in water and this solution will spread over the remaining granules, as a result of which the indicators will also spread over a larger area and the colour indication will therefore be more difficult to observe with the eye.

Another drawback of the achievement as revealed in European Patent Application EP 0 569 232 is that the granules are in direct contact with the air and therefore in direct contact with the carbonic acid in the air, which also affects the measurement of the degree of acidity, since carbonic acid gas forms an acid with moisture. As a result, the measurements will also be adversely affected.

The object of the invention is an indicator with which the pH or degree of acidity of the urine of a cat can easily be determined and in which the abovementioned disadvantages do not occur.

This object of the invention is achieved in that the means comprise a continuous envelope or cell having a liquid-permeable wall, a certain amount of indicator material being enclosed in the envelope.

The advantage of using such an indicator is:
- that the indicator material does not come into or is not in direct contact with the products, such as clay granules, with which the cat tray is filled, so that basic or acidic components of the cat tray filling cannot diffuse into the indicator material;
- that the indicator material also does not come into direct contact with the air, as a result of which carbonic acid from the air can hardly affect the measurement of the degree of acidity;
- that, after the indicator means have absorbed the moisture or urine, the moisture absorbed cannot readily evaporate from the envelope, as a result of which the colour of the indicator powder continues to be maintained for a long time.

In an exemplary embodiment of the invention, the envelope comprises a first layer and a second layer which is attached at the edges over the entire circumference to the edges of the first layer, for example glued or sealed, indicator material being enclosed between the first layer and the second layer and the envelope being manufactured at least partially from a liquid-permeable material, at least a part of the envelope being transparent. Such an indicator is advantageous to manufacture. The indicator material is well protected from the environment because moisture can readily be absorbed by the indicator material through the liquid-permeable first strip.

The first layer or the second layer or both are preferably manufactured from two outermost strips of a transparent plastics-material film and from a central liquid-permeable strip of a hydrophilized plastics material. The colour change can consequently readily be observed through the transparent strip. The moisture is attracted by the hydrophilized plastics material of the central strip so that the indicator material is readily moistened.

In this connection, there is preferably disposed between the first and the second layer an interlayer of a plastics material having an open fibre structure, such as an open nonwoven plastics material or a textile plastics material which are composed in both cases of, for example, polyethylene terephthalate, polypropylene, polyamide or viscose, the indicator material being powdered and being absorbed in the third layer. Said interlayer keeps the first and second layer apart and fixes the preferably powdered indicator material. The fixing is promoted by first mixing the powder with solvent and then applying it to the interlayer and then evaporating the solvent.

Preferably, the central, liquid-permeable strip of the first and second layers is manufactured by means of pressing randomly aligned fibres or threads of a hydrophilized plastics material, or continuous nonwoven plastics material, such as polyethylene terephthalate, polypropylene, polyamide or viscose. As a result, a porous zone is obtained which attracts moisture well and then allows it through to the interior of the envelope.

In another possible embodiment of the invention, the envelope comprises a capsule whose wall is provided with a number of small openings. The shape of the capsule can be chosen so that it corresponds to the shape of a cat tray granule so that the cat is not frightened by it. The wall of the capsule allows liquid through via the small holes made in the wall and protects the indicator material in an expedient manner from the environmental influences.

In order to be able to allow the moisture readily to penetrate the envelope, the capsule is preferably manufactured from a hydrophilic, transparent material which can readily be moistened, such as polyethylene terephthalate, polyvinyl alcohol or polyurethane. As a result, the moisture will readily be able to penetrate the capsule and, in addition, the colour can readily be observed through the wall of the capsule. The capsule can be manufactured in an easy and advantageous way from said materials by means of injection moulding.

Preferably, the indicator material is composed of a powder, the particle size of the powder being less than 1.0 mm. The particle size distribution is important for a good moistening of the indicator. The fine material present in the powder collects in the small holes in the envelope without leaving the envelope. As a result, an accelerated moistening of the indicator powder occurs.

Preferably, the indicator material contains one or more suitable indicators and a material which swells as a result of absorbing moisture. When moistened with the urine, the powder swells until the entire envelope is filled, so that the colour of the indicator material can readily be observed. For a more rapid and better moistening of the indicator material, the indicator material also comprises a material which accelerates the transport of moisture or transport of urine.

The powdered indicator material is preferably composed of a ground or dissolved and then dried mixture of bromothymol blue, alizarin, carboxymethylcellulose and cellulose. As a result, an environmentally friendly pH indicator is obtained whose colour changes at a pH between 6.5 and 7.0. The colour change associated with this is from yellow at a pH of 6.0 to blue/green at a pH of 7.0. It has been found that this colour change remains readily visible for approximately 24 hours after being moistened with urine.

The invention will be explained in greater detail by reference to the drawing. In the drawing:
- Figure 1: shows a view of a closed capsule according to the invention;
- Figure 2: shows a view of the two parts of the capsule according to Figure 1;
- Figure 3: shows a cross section through a capsule according to Figure 1;
- Figure 4: shows a view of an indicator according to the invention;
- Figure 5: shows a cross section along the line V-V according to Figure 4;
- Figure 6: shows an indicator according to Figure 4 after moistening.

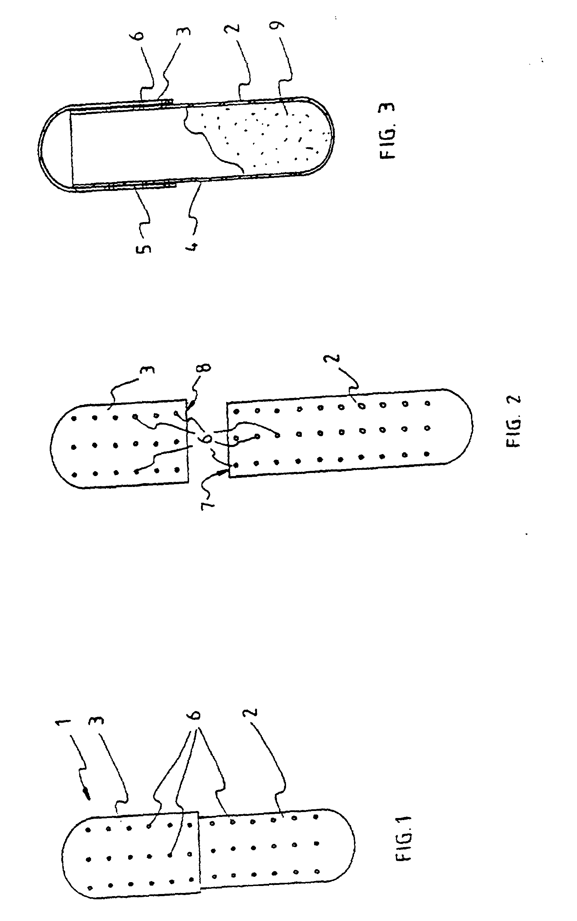

Figures 1 and 2 show in elevation a cylindrical capsule 1 according to the invention, while Figure 3 shows the same capsule 1 from Figures 1 and 2 in cross section. The capsule 1 comprises a body 2 and a cover or lid 3. Openings or small holes 6 have been made in the walls 4, 5 of the two parts 2, 3 of the capsule 1. The two parts 2, 3 of the capsule 1 are manufactured by means of injection moulding from a hydrophilic, transparent plastics material.

Figure 2 shows the two parts 2, 3 of the capsule 1 detached from each other. The open end 7 of the body 2 can be pushed into the open end 8 or the cover 3 in a clamping manner. Preferably, the small holes o have been made in the walls 4 and 5 of the body 2 and the cover 3 in such a way that, if the two parts have been pushed onto each other, the small holes correspond to each other and are situated above each other in line with each other so that each small hole 6 in the cover 3 is in communication with a small hole 6 of the body 2. For this purpose, the shape of the two parts 2, 3 of the capsule is preferably made such that, if the cover has been pushed completely onto the body, the small holes correspond to each other. If necessary, the outside edge of the body 2 and the inside wall of the cover 3 are provided for this purpose with grooves, or for example, are of somewhat angular design so that the cover 3 can be placed on the body 2 only in one way.

Both components 2, 3, the body 2 and the closure cover 3 of the capsule 1 are manufactured from a transparent and easy-to-moisten material, such as polyethylene terephthalate, polyvinyl alcohol or polyurethane. Because the capsule 1 is transparent, a colour change of the indicator material can easily and clearly be observed from the outside.

Figure 3 shows the capsule 1 in cross section. The body 2 contains a powdered material 9. Said powdered material 9 is a mixture of a powdered indicator material together with a powdered swelling material which, when it absorbs moisture, swells and substantially fills the open space in the capsule 1 so that, after absorbing the moisture, the colour of the indicator material is readily visible through the transparent outside wall 4 of the capsule 1. The swelling material used may be composed, for example, of cellulose and/or carboxymethylcellulose.

The action is as follows:

A number of capsules are mixed with the cat tray granules in a cat tray. As soon as the cat urinates on the cat tray, the moisture will penetrate via the openings 6 into one or more capsules and reach the mixture of indicator material and swelling material 9 enclosed in the capsules 1. The openings 6 are approximately 0.1 to 0.3 mm in diameter preferably 0.2 mm, so that the openings 6 are large enough readily to allow the urine of a cat through, while they are small enough, on the other hand, to prevent the indicator material, which is composed of particles smaller than 1 mm, from being able to come out. The openings 6 are uniformly distributed over the walls of the capsule 1. In the exemplary embodiment according to the figures, the openings 6 are in rows. In another possible embodiment, the openings 6 can be arranged helically.

After the moisture has penetrated the capsule 1, the indicator material will assume the colour which is associated with the degree of acidity of the moisture and the swelling material will expand as a result of absorbing moisture and fill the space in the capsule. Because the indicator material is uniformly distributed over the swelling material, the colour will easily be capable of being observed through the transparent wall of the capsule.

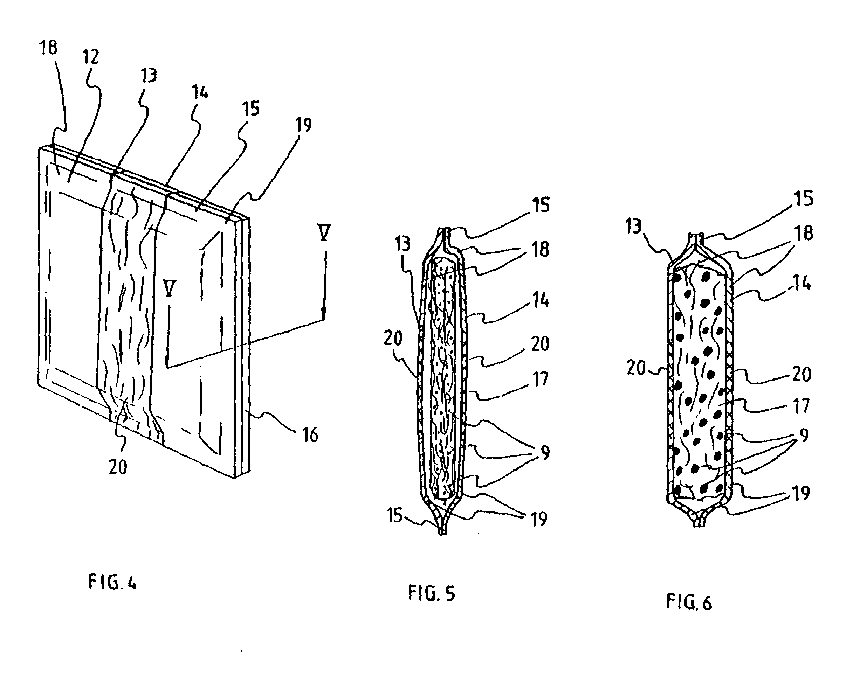

Figure 4 shows another possible embodiment of an indicator according to the invention, which is shown in cross section in Figure 5. Said indicator comprises an envelope 12 comprising a first thin layer or film 13 and an identical second layer or film 14. The first layer 13 is attached, for example sealed or glued, at the edges 15 over the entire circumference to the edges 16 of the transparent second layer 14. Between the first layer 13 and the second layer 14 there is a third layer 17 which is composed of an open nonwoven plastics material. Said layer 17 has an open structure in which a powdered indicator material 9 is uniformly distributed. The third layer 17 fixes the powdered indicator material 9 and keeps the first layer 13 and the second layer 14 at a mutual distance. The indicator is approximately 2 to 4 mm thick. Both the first layer 13 and the second layer 14 are constructed of three strips placed alongside one another, namely two outermost strips 18, 19 and a central strip 20. The outermost strips 18, 19 have been manufactured from a transparent plastics-material film, for example transparent polyethylene film. The central strip 20 has been manufactured from a hydrophilic, liquid-permeable plastics material, for example closed nonwoven plastics material, unaligned plastics-material fibres being pressed onto one another so that a porous film is produced.

The action of the indicator constructed in this way is similar to the action of the capsule from Figures 1 to 3 inclusive. When moisture, such as the urine of a cat, reaches the indicator, the moisture enters the indicator via the liquid-permeable strip 20 and moistens the indicator material 9. This will become coloured depending on the degree of acidity of the liquid and, in addition, will swell. In this process, the indicator becomes approximately twice as thick. As a result of the swelling, the indicator material is pressed against the layers 13, 14 so that the colour of the indicator material can clearly be observed through the transparent strips 18, 19.

Figure 6 shows in cross section the indicator after it has been moistened with, for example, the urine of a cat. The indicator material 9 in the interlayer 17 has swollen, as a result of which the indicator has become approximately twice as thick. The indicator material 9 assumes a colour which is dependent on the degree of acidity of the moisture absorbed. This colour can be clearly observed through the transparent strips 18, 19 because the indicator material is pressed against said strips 18, 19 as a consequence of the swelling.

## Claims

1. Means for determining the pH of a liquid, such as the urine of a cat, with the aid of colour change **characterized in that** the means comprise a continuous envelope (1; 12) or cell having a liquid-permeable wall (4, 5; 13, 14), a certain amount of indicator material (9) being enclosed in the envelope (1; 12).

2. Means according to Claim 1, **characterized in that** the envelope (12) comprises a first layer (13) and a second layer (14) which is attached at the edges (16)over the entire circumference to the edges (15) of the first layer (13), indicator material (9) being enclosed between the first layer (13) and the second layer (14) and the envelope (12) being manufactured at least partially from a liquid-permeable material (20), at least a part of the envelope (12) being transparent.

3. Means according to Claim 2, **characterized in that** the first layer (13) or the second layer (14) or both (13, 14) are manufactured from two outermost strips (18, 19) of a transparent plastics-materials film and from a central liquid-permeable strip (20) of a hydrophilized plastics material.

4. Means according to Claim 2 or 3, **characterized in that** there is disposed between the first (13) and the second (14) layer a third layer (17) or an interlayer of a plastics material having an open fibre structure or an open nonwoven plastics material, the indicator material (9) being powdered and being absorbed in the third layer (17).

5. Means according to claim 3, **characterized in that** the central, liquid-permeable strip (20) of the first (13) and second (14) layers is manufactured by means of pressing randomly aligned fibres or threads of a hydrophilized plastics material, or continuous nonwoven plastics material, such as polyethylene terephthalate, polypropylene, polyamide or viscose.

6. Means according to Claim 1, **characterized in that** the envelope (1) comprises a capsule (1) whose wall (4, 5) is provided with a number of small openings (6).

7. Means according to Claim 6, **characterized in that** the capsule (1) is manufactured from a hydrophilic, transparent material which can readily be moistened.

8. Means according to Claim 7, **characterized in that** the capsule (1) is manufactured from a transparent hydrophilic polymer.

9. Means according to one of the preceding claims, **characterized in that** the indicator material (9) contains one or more suitable indicators and a material which swells as a result of absorbing moisture.

10. Means according to one of the preceding claims, **characterized in that** the indicator material (9) also comprises a material which accelerates the transport of moisture or the transport of urine.

11. Means according to Claim 9, **characterized in that** the swelling material is composed of cellulose and/or carboxymethylcellulose.

12. Means according claim 6, 7 or 8, **characterized in that** at least a part of the wall (3, 4) of the envelope (1) comprises openings (6) having a diameter which is less than 0.5 mm.

13. Means according to Claim 12, **characterized in that** the diameter of the openings (6) is between 0.1 and 0.3 mm.

14. Means according to one of the claims 6, 7, 8, 12 or 13, **characterized in that** openings (6) have been made which are uniformly distributed over the outside surface of the envelope (1).

15. Means according to one of the preceding Claims 6 to 14 inclusive, **characterized in that** the capsule (1) comprises a body (2) and a closure cover (3), the openings (6) in the closure cover (3) corresponding to the openings (6) in the body (2).

16. Means according to one of the preceding claims, **characterized in that** the openings (6) have been made in a helical pattern.

17. Means according to one of the preceding claims, **characterized in that** the indicator material (9) is composed of a powder, the particle size of the powder being less than 1.0 mm.

18. Means according to Claim 17, **characterized in that** the powder is composed of a ground mixture of bromothymol blue, alizarin, carboxymethylcellulose and cellulose.

19. Means according to claim 4, **characterized in that** said plastics material is chosen form the group consisting of polyethylene terephthalate, polypropylene, polyamide or viscose.

20. Means according to claim 8, **characterized in that** said transparent hydrophilic polymer is chosen form the group consisting of polyethylene terephthalate, polyvinyl alcohol, polyurethane.

## Patentansprüche

1. Mittel zur Bestimmung des pH-Wertes einer Flüssigkeit, wie Katzenurin, mit Hilfe einer Farbveränderung, **dadurch gekennzeichnet, daß** die Mittel eine geschlossene Umhüllung (1; 12) oder Zelle umfassen, mit einer flüssigkeitsdurchlässigen Wand (4, 5; 13, 14), wobei in der Umhüllung (1; 12) eine bestimmte Menge von Indikatormaterial (9) aufgeschlossen ist.

2. Mittel nach Anspruch 1, **dadurch gekennzeichnet, daß** die Umhüllung (12) eine erste Schicht (13) und eine an den Rändern (16) über den gesamten Umfang an den Rändern (15) der ersten Schicht (13) befestigten zweite Schicht (14) umfaßt, wobei zwischen der ersten Schicht (13) und der zweiten Schicht (14) Indikatormaterial (9) aufgeschlossen ist und wobei die Umhüllung (12) zumindest teilweise aus einem flüssigkeitsdurchlässigen Material (20) hergestellt ist, wobei zumindest ein Teil der Umhüllung (12) durchsichtig ist.

3. Mittel nach Anspruch 2, **dadurch gekennzeichnet, daß** die erste Schicht (13) oder die zweite Schicht (14) oder beide (13, 14) aus zwei äußeren Streifen (18, 19) aus einer durchsichtigen Kunststofffolie und aus einem mittleren flüssigkeitsdurchlässigen Streifen (20) aus einem hydrophilisierten Kunststoff hergestellt sind.

4. Mittel nach Anspruch 2 oder 3, **dadurch gekennzeichnet, daß** zwischen der ersten (13) und der zweiten (14) Schicht eine dritte Schicht (17) oder eine Zwischenschicht aus einem Kunststoff mit einer offenen Faserstruktur oder aus einem offenen Nonwoven Kunststoff angeordnet ist, wobei das Indikatormaterial (9) pulverförmig ist und in der dritten Schicht (17) aufgenommen ist.

5. Mittel nach Anspruch 3, **dadurch gekennzeichnet, daß** der mittlere flüssigkeitsdurchlässige Streifen (20) der ersten (13) und zweiten (14) Schichte durch Pressen von beliebigen gerichteten Fasern oder Faden aus einem hydrophilisierten Kunststoff, oder einem geschlossenen Nonwoven Kunststoff, wie zum Beispiel Polyethylenterephthalat, Polypropylen, Polyamid oder Viskose hergestellt ist.

6. Mittel nach Anspruch 1, **dadurch gekennzeichnet, daß** die Umhüllung (1) aus einer Kapsel (1) besteht, deren Wand (4, 5) mit einer Anzahl kleine Öffnungen (6) versehen ist.

7. Mittel nach Anspruch 6, **dadurch gekennzeichnet, daß** die Kapsel (1) aus einem einfach zu befeuchtenden, hydrophilen, durchsichtigen Material hergestellt ist.

8. Mittel nach Anspruch 7, **dadurch gekennzeichnet, daß** die Kapsel (1) aus einem transparanten hydrophilen Polymer hergestellt ist.

9. Mittel nach einem der vorhergehenden Ansprüche, **dadurch gekennzeichnet, daß** das Indikatormaterial (9) einen oder mehrere geeigneten Indikatore und ein Material, das durch die Aufnahme von Feuchte quellt, umfaßt.

10. Mittel nach einem der vorhergehenden Ansprüche, **dadurch gekennzeichnet, daß** das Indikatormaterial (9) zugleich ein den Feuchtetransport oder Urintransport verschnellendes Material umfaßt.

11. Mittel nach Anspruch 9, **dadurch gekennzeichnet, daß** das Quellmaterial aus Zellulose und/oder Carboxymethylzellulose besteht.

12. Mittel nach Anspruch 6, 7 oder 8, **dadurch gekennzeichnet, daß** zumindest ein Teil der Wand (3, 4) der Umhüllung (1) Öffnungen (6) mit einem Diameter kleiner als 0,5 mm umfaßt.

13. Mittel nach Anspruch 12, **dadurch gekennzeichnet, daß** der Diameter der Öffnungen (6) zwischen 0,1 und 0,3 mm ist.

14. Mittel nach einem der Ansprüche 6, 7, 8, 12 oder 13, **dadurch gekennzeichnet, daß** gleichmäßig über die Außenfläche der Umhüllung (1) verteilte Öffnungen (6) angeordnet sind.

15. Mittel nach einem der vorhergehenden Ansprüche 6 bis einschließlich 14, **dadurch gekennzeichnet, daß** die Kapsel (1) einen Körper (2) und eine Verschlußkappe (3) umfaßt, wobei die Öffnungen (6) in der Verschlußkappe (3) mit den Öffnungen (6) im Körper (2) übereinstimmen.

16. Mittel nach einem der vorhergehenden Ansprüche, **dadurch gekennzeichnet, daß** die Öffnungen (6) in einer schraubenförmigen Struktur angeordnet sind.

17. Mittel nach einem der vorhergehenden Ansprüche, **dadurch gekennzeichnet, daß** das Indikatormaterial (9) aus einem Pulver besteht, wobei die Teilchengröße des Pulvers kleiner als 1,0 mm ist.

18. Mittel nach Anspruch 17, **dadurch gekennzeichnet, daß** das Pulver aus einer pulverisierten Mischung von Bromothymolblau, Alizarin, Carboxymethylzellulose und Zellulose besteht.

19. Mittel nach Anspruch 4, **dadurch gekennzeichnet, daß** der Kunststoff aus der Gruppe bestehend aus Polyethylenterephthalat, Polypropylen, Polyamid oder Viskose ausgewählt ist.

20. Mittel nach Anspruch 8, **dadurch gekennzeichnet, daß** das durchsichtige hydrophile Polymer aus der Gruppe bestehend aus Polyethylenterephthalat, Polyvinyl Alkohol, Polyurethan ausgewählt ist.

## Revendications

1. Moyen permettant de déterminer le pH d'un liquide, tel que l'urine d'un chat, à l'aide d'un changement de couleur **caractérisé en ce que** le moyen comprend une enveloppe continue (1; 12) ou une cellule ayant une paroi perméable aux liquides (4, 5; 13, 14), une certaine quantité de matériau indicateur (9) étant enfermée dans l'enveloppe (1; 12).

2. Moyen selon la revendication 1, **caractérisé en ce que** l'enveloppe (12) comprend une première couche (13) et une seconde couche (14) qui est attachée au niveau des bords (16) sur la circonférence entière aux bords (15) de la première couche (13), un matériau indicateur (9) étant enfermé entre la première couche (13) et la seconde couche (14) et l'enveloppe (12) étant fabriquée au moins partiellement à partir d'un matériau perméable aux liquides (20), au moins une partie de l'enveloppe (12) étant transparente.

3. Moyen selon la revendication 2, **caractérisé en ce que** la première couche (13) ou la seconde couche (14) ou les deux (13, 14) sont fabriquées à partir de deux bandes les plus extérieures (18, 19) d'un film de matière plastique transparent et à partir d'une bande perméable aux liquides centrale (20) d'une matière plastique rendue hydrophile.

4. Moyen selon la revendication 2 ou 3, **caractérisé en ce qu'**on a disposé entre la première (13) et la seconde (14) couche une troisième couche (17) ou une couche intermédiaire d'une matière plastique ayant une structure à fibres ouverte ou d'une matière plastique non tissée ouverte, le matériau indicateur (9) étant en poudre et absorbé dans la troisième couche (17).

5. Moyen selon la revendication 3, **caractérisé en ce que** la bande perméable aux liquides centrale (20) des première (13) et seconde (14) couches est fabriquée par pressage de filaments ou de fibres alignés au hasard d'une matière plastique rendue hydrophile, ou d'une matière plastique non tissée continue, telle que le poly(téréphtalate d'éthylène), le polypropylène, le polyamide ou la viscose.

6. Moyen selon la revendication 1, **caractérisé en ce que** l'enveloppe (1) comprend une capsule (1) dont la paroi (4, 5) est dotée d'un certain nombre de petites ouvertures (6).

7. Moyen selon la revendication 6, **caractérisé en ce que** la capsule (1) est fabriquée à partir d'un matériau transparent, hydrophile, qui peut facilement être humidifié.

8. Moyen selon la revendication 7, **caractérisé en ce que** la capsule (1) est fabriquée à partir d'un polymère hydrophile transparent.

9. Moyen selon l'une quelconque des revendications précédentes, **caractérisé en ce que** le matériau indicateur (9) contient un ou plusieurs indicateurs appropriés et un matériau qui gonfle par suite de l'absorption d'humidité.

10. Moyen selon l'une quelconque des revendications précédentes, **caractérisé en ce que** le matériau indicateur (9) contient également un matériau qui accélère le transport d'humidité ou le transport d'urine.

11. Moyen selon la revendication 9, **caractérisé en ce que** le matériau gonflant est composé de cellulose et/ou de carboxyméthylcellulose.

12. Moyen selon la revendication 6, 7 ou 8, **caractérisé en ce qu'**au moins une partie de la paroi (3, 4) de l'enveloppe (1) comprend des ouvertures (6) ayant un diamètre qui est inférieur à 0,5 mm.

13. Moyen selon la revendication 12, **caractérisé en ce que** le diamètre des ouvertures (6) est compris entre 0,1 et 0,3 mm.

14. Moyen selon l'une quelconque des revendications 6, 7, 8, 12 ou 13, **caractérisé en ce que** des ouvertures (6) ont été faites qui sont uniformément réparties sur toute la surface extérieure de l'enveloppe (1).

15. Moyen selon l'une quelconque des revendications 6 à 14 incluses, **caractérisé en ce que** la capsule (1) comprend un corps (2) et un couvercle de fermeture (3), les ouvertures (6) dans le couvercle de fermeture (3) correspondant aux ouvertures (6) dans le corps (2).

16. Moyen selon l'une quelconque des revendications précédentes, **caractérisé en ce que** les ouvertures (6) ont été faites suivant un motif hélicoïdal.

17. Moyen selon l'une quelconque des revendications précédentes, **caractérisé en ce que** le matériau indicateur (9) se compose d'une poudre, la taille de particule de la poudre étant inférieure à 1,0 mm.

18. Moyen selon la revendication 17, **caractérisé en ce que** la poudre se compose d'un mélange broyé de bleu de bromothymol, d'alizarine, de carboxyméthyl-cellulose et de cellulose.

19. Moyen selon la revendication 4, **caractérisé en ce que** ladite matière plastique est choisie dans le groupe constitué du poly(téréphtalate d'éthylène), du polypropylène, du polyamide ou de la viscose.

20. Moyen selon la revendication 8, **caractérisé en ce que** ledit polymère hydrophile transparent est choisi dans le groupe constitué du poly(téréphtalate d'éthylène), de l'alcool polyvinylique, du polyuréthane.
